Europäisches Patentamt

European Patent Office

Office européen des brevets

(19)

(11) Publication number: **0 070 164**
**B1**

# EUROPEAN PATENT SPECIFICATION

(45) Date of publication of patent specification: **24.09.86**

(51) Int. Cl.⁴: **D 04 H 1/54,** A 61 F 13/16

(21) Application number: **82303606.6**

(22) Date of filing: **09.07.82**

(54) Absorbent nonwoven fabric containing staple length polyester/polyethylene conjugate fibers and absorbent fibers.

(30) Priority: **10.07.81 US 282325**
**07.12.81 US 327836**
**02.06.82 US 383260**

(43) Date of publication of application:
**19.01.83 Bulletin 83/03**

(45) Publication of the grant of the patent:
**24.09.86 Bulletin 86/39**

(84) Designated Contracting States:
**AT DE FR GB IT NL SE**

(56) References cited:
**GB-A-1 149 270**
**US-A-3 511 747**
**US-A-3 713 875**
**US-A-4 023 570**

(73) Proprietor: **CHICOPEE**
**317 George Street**
**New Brunswick, New Jersey 08903 (US)**

(72) Inventor: **Fekete, Paul**
**22, Watchung Road**
**East Brunswick New Jersey 08816 (US)**
Inventor: **Mays, Alfred Thomas**
**6 Lynnfield Drive**
**East Windsor New Jersey 08520 (US)**

(74) Representative: **Jones, Alan John et al**
**CARPMAELS & RANSFORD 43 Bloomsbury Square**
**London, WC1A 2RA (GB)**

Courier Press, Leamington Spa, England.

# 0 070 164

## Description

The invention relates to a low density, highly absorbent, thermal bonded nonwoven fabric comprising absorbent fibers and staple length polyethylene/polyester conjugate fibers, and to a process for making said nonwoven fabric.

Absorbant low density nonwoven fabrics have wide utility for use in catamenial devices such as panty shields, in absorbent pads and bandages, as wipes, and in similar applications. There is continuing interest in producing such nonwoven fabrics having higher absorption capacity and in producing such fabrics by methods that use less energy. The present invention provides such a nonwoven fabric having very high absorbent capacity, and which can be made by using a minimum of energy.

Samejima, in US—A—4,160,159, discloses an absorbent fabric containing wood pulp and short length heat-fusible fibers, said fabric being supported on a web such as tissue paper. The web also contains powdered absorbent material distributed throughout. In producing the fabric, the heat-fusible fibers are fused, and thus the integrity of the heat-fusible fibers is destroyed.

Marshall, in US—A—4,083,913, also discloses a nonwoven fabric produced from short length heat-fusible fibers and absorbent fibers, wherein, again, in producing the final fabric, the heat-fusible fibers are fused and destroyed.

Other patents showing nonwoven fabrics made from absorbent fibers and fusible fibers include Secrist, US—A—2,774,128, Wolterding et al., US—A—3,067,747, and Tsuchiya et al., US—A—4,307,721.

US—A—3 511 747, Davies, discloses a bonded textile material containing fibres consisting of at least two fibre forming synthetic polymer components arranged in distinct zones across the cross-section of each fiber, at least one, but not all of which components are potentially adhesive and located on at least a portion of the peripheral surface thereof. The fibers are bonded by the adhesive material, but do not form crimps during the formation of the mat due to the application of pressure through bonding.

The fabric may comprise staple length or blends of staple length fibers or wet laid mats of pulp length fibers. The fabrics made from pulp length fibers have a density of 0.25 g/cm³.

The fabric of the invention is a low density, highly absorbent, thermal bonded nonwoven fabric comprising pulp length absorbant fibers and staple length polyester/polyethylene conjugate fibers.

The nonwoven fabrics of the invention are produced by a process which comprises:

(a) producing a web comprising pulp length absorbent fibers and staple length polyester/polyethylene conjugate fibers;

(b) subjecting said web to a temperature sufficient to fuse the lower melting component of said conjugate fibers without fusing the higher melting component of said conjugate fibers, while maintaining said web under little or no compression; and

(c) cooling said web to resolidify the lower melting component of said conjugate fibers, to thereby form bonds at the sites where said conjugate fibers touch other fibers.

Brief description of the drawings

Fig. 1 is a schematic side elevation of an apparatus suitable for carrying out the process of the invention;

Fig. 2 is a perspective view of a catamenial device (panty shield) made from the nonwoven fabric of the invention;

Figs. 3 and 4 are perspective views of a wiping cloth made from the nonwoven fabric of the invention;

Figs. 5 and 6 are perspective views of a wound dressing made from the nonwoven fabric of the invention; and

Fig. 7 is a cross-section taken along line 7—7 of Fig. 5.

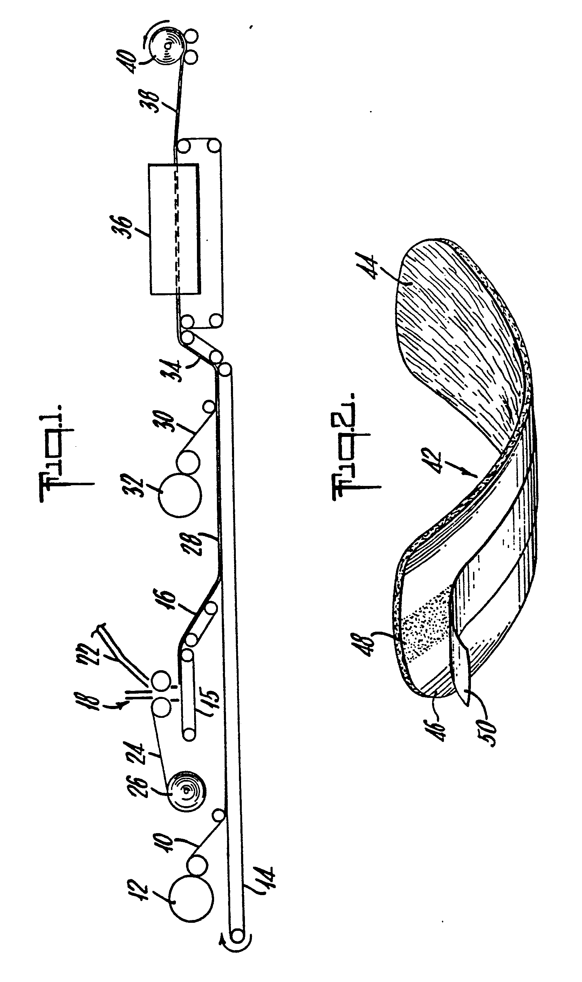

Referring first to Fig. 1, one preferred arrangement of apparatus for carrying out the process of the invention is displayed. The apparatus shown in Fig. 1 is suitable for making a nonwoven fabric of the invention comprising a core of a mixture of short-length (pulp length) natural cellulose fibers as the absorbent fibers and staple length polyester/polyethylene conjugate fibers, with a light weight veneer comprising heat-fusible fibers on both faces of the fabric. A light weight web 10 of heat-fusible fibers is laid down, as from a card 12, on a first endless belt 14. A second web 16 comprising a mixture of short-length (pulp length) natural cellulose fibers and staple length polyester/polyethylene conjugate fibers is laid on a second endless belt 15. The second web 16 is produced in an air laying apparatus, for instance a dual rotor 18, such as is described by Ruffo and Goyal in US—A—3,768,118. The feed to the dual rotor 18 includes partially opened staple length polyester/polyethylene conjugate fibers fed through a chute feed 22, and a web of pulp sheet 24 composed of compacted wood pulp fibers, from a feed roll 26. The said second web 16 is fed onto the top of the light weight web 10, to form a double layer web 28.

The double layer web 28 containing the web of heat-fusible fibers 10 on the bottom and the mixed web 16 on the top is then passed under another station wherein a second light weight web of heat-fusible fibers 30 is laid on top, as from a card 32. The resulting triple layer web 34 is then passed through a through-air dryer 36 to fuse the lower melting component of the conjugate fibers while maintaining the integrity of these fibers as fibers, and to fuse or soften the surfaces of the heat-fusible fibers in the two veneers 10 and 30. When the triple layer web emerges from the dryer 36, it cools to thereby form the non-woven fabric 38 of the invention. When the web 36 cools, the fused surfaces of the heat-fusible fibers, and the fused lower

2

melting component of the conjugate fibers, i.e. the polyethylene, solidify, and bonds then form where these surfaces touched other fibers. (By "other" fibers, is meant other heat-fusible and conjugate fibers as well as other types of fibers that may be present.) The fabric 38 is then collected on a conventional windup 40.

The thermal bonded nonwoven fabrics of the invention employ polyester/polyethylene conjugate fibers wherein at least about 50 per cent of the surface of the individual fibers is polyethylene. It is preferred to employ sheath/core fibers with the polyethylene as the sheath and the polyester as the core. Either eccentric or concentric sheath/core fibers can be employed. The fibers will usually have a denier within the range of from about 1 to about 6 (0.11 to 0.66 Tex), and are usually in excess of about 1/2-inch (1.27 cm) in length, up to about 3 or 4 inches (7.62 or 10.16 cm) long.

Preferably, the conjugate fibers employ high density polyethylene, that is linear polyethylene that has a density of at least about 0.94, and a Melt Index ("M.I.") by ASTM D-1238(E) (190°C, 2160 g) of greater than 1, preferably greater than about 10, and more preferably from about 20 to about 50. Usually the fibers will be composed of about 40 to 60 weight per cent, and preferably 45 to 55 weight per cent, polyester, the remainder being polyethylene.

The thermal bonded nonwoven fabrics of the invention may also employ absorbent fibers, such as rayon staple fibers or cotton fibers, in addition to the pulp length absorbent fibers, such as short length natural cellulose fibers including wood pulp fibers and cotton linters and mixtures thereof.

The pulp length absorbent fibers and the conjugate fibers are blended and formed into the feed web (e.g. 16 in Fig. 1) by standard methods. When the absorbent fibers include short length natural cellulose fibers, it is preferred to form the web by air laying, as by the use of the dual rotor disclosed, for instance, by Ruffo et al., in US—A—3,768,118.

When a veneer of heat-fusible fibers is used, it is preferred that said heat-fusible fibers also be staple length conjugate fibers. However, if desired, other types of heat-fusible fibers such as polypropylene homofil fibers, can be used in the veneer. The veneer can also contain other fibers, such as rayon, cotton, or polyester staple fibers, that are not ordinarily heat-fusible.

The proportion of the polyester/polyethylene conjugate fibers in the web can vary from about 15 percent to about 75 percent (based on weight of conjugate fibers plus absorbent fibers), depending upon the end use intended for the fabric. The exact proportion has not been found to be narrowly critical.

If desired, other fibers can be included in the web, such as staple length polyester fibers, polypropylene fibers, and other known types of fibers.

The fabrics of the invention normally have basis weights from about 1/2 to about 6 ounces per square yard (17 to 203 g/m²). The bulk density of the fabrics of the invention is usually below about 0.15 gram per cubic centimeter, preferably below about 0.09 gram per cubic centimeter, e.g. from about 0.02 to about 0.09 gram per cubic centimeter, and more preferably from about 0.025 to about 0.06 gram per cubic centimeter. The fabrics preferably have an absorbent capacity, as measured by a Gravimetric Absorbency Tester (described below, in the Examples), of at least 600 per cent and preferably at least 1400 percent, exclusive of any non-absorbent layer such as a veneer of 100 per cent fusible fibers. The excellent resistance of the fabrics to wet collapse contributes to this high absorbent capacity. The bulk density and absorbent capacity figures disclosed here refer to the initial thermal bonded fabric, prior to any post treatment such as hot embossing, which can reduce the bulk density of the fabric.

The web of the invention is thermal bonded under conditions such that the integrity of the conjugate fibers is maintained. The purpose of maintaining the integrity of these fibers is to maintain the high loft or low bulk density characteristics of the finished nonwoven fabric, in order to achieve optimum absorption capacity and strength. The web is thermal bonded under conditions of zero pressure, or very light pressure, so that the web is not significantly crushed or compacted while the thermal bonding step is being carried out. The exact temperatures employed in the thermal bonding will vary, depending upon the weight and bulk density of the web, and the dwell time employed in the heated zone. The bonding temperature is usually from about 100° to about 150°C. Dwell times in the heated zone will usually vary from about 2 seconds to about one minute. Specific conditions under which the thermal bonding is achieved are illustrated in the examples below. The temperatures referred to are the temperatures to which the fibers are heated in order to achieve bonding. In order to achieve high speed operations, much higher temperatures with short exposure times can be used. Routine experimentation will suffice to determine practical conditions.

In the thermal bonding step, the polyethylene, which is the lower melting component of the conjugate fiber, is at least partially fused so that where the fused surface touches another fiber, especially the fused surface of another conjugate fiber, welding or fusing together of the two fibers will occur. It is an important feature of this invention that the conjugate fibers remain fibers because the polyester does not melt, shrink, curl, or change its orientation during the thermal bonding step. This enables the thermal bonded fabric to maintain the bulk of the unbonded feed web.

The thermal bonding step can be carried out by through-air bonding, as is illustrated in Fig. 1, or by other means such as infrared heating or other type of radiant heating. Through-air heating can be accomplished by carrying the web on a porous conveyor belt through a zone where hot air is forced through the web, it can be carried through a heated zone contained between two porous belts, or it can be carried around a rotating drum having a porous surface which is equipped to suck hot air through the web as it is passing around the drum. The exact method of effecting the heating has not been found to be narrowly critical.

After the above-described thermal bonding, additional bonding and/or treatment of the web can be carried out. For instance, the fabric can be hot embossed to improve the overall integrity of the fabric, especially the surface abrasion resistance, to enhance utility in certain areas such as wiping.

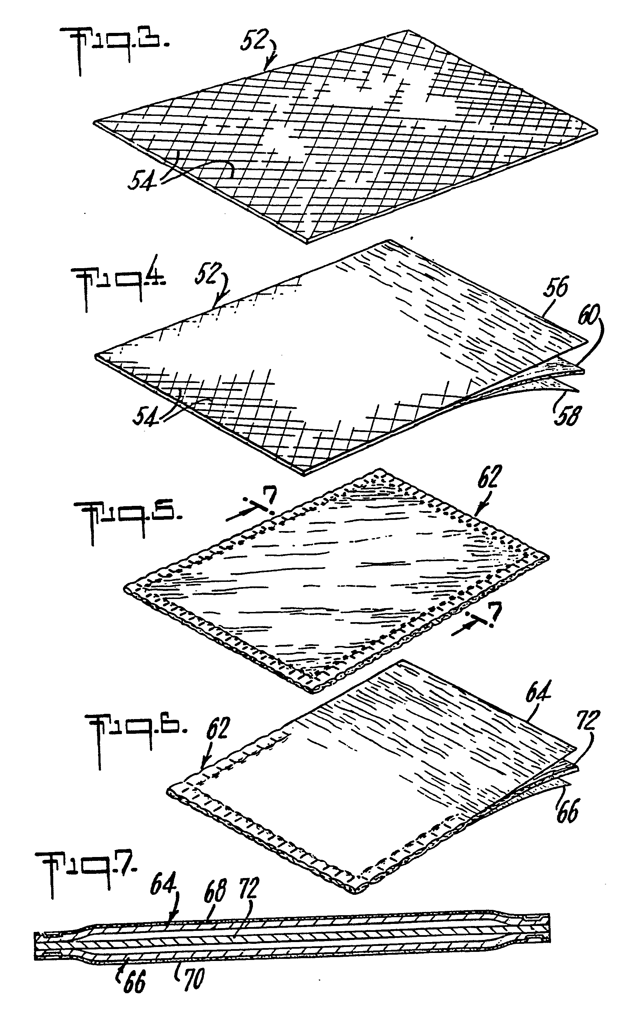

In Fig. 2, a panty shield 42 made of the non-woven fabric of the invention is shown. Such panty shields are described in US—A—4,023,570 and US—A—4,023,571.

The examples below illustrate various aspects of the invention.

Example 1

Using an apparatus similar to that shown in Fig. 1, a veneered fabric was made from wood pulp and Fiber A, a 2.5—3 denier (0.28—0.33 Tex) bicomponent fiber, 1.5 inches (3.81 cm) long, having a high density polyethylene sheath and a polyester core, with the two polymers being present in a weight ratio of about 50/50. The polyethylene has a melt index of 42, a density of 0.96, and a DSC melting point of 132°C. The polyester is fiber grade polyethylene terephthalate. The fibers have a tenacity of about 3.3 grams per denier (0.37 g/Tex), an elongation of 30 to 60 per cent, and from 12 to 18 crimps per inch (crimps per 2.54 cm). The core was composed of about 70 weight per cent pulp fiber and 30 weight per cent Fiber A, and the two veneers were composed of Fiber A. One veneer weighed 160 grains per square yard (12.4 g/m$^2$), the other weighed 90 grains per square yard (7.0 g/m$^2$), and the entire fabric weighed 2.2 ounces per square yard (74.6 g/m$^2$). The fabric was through-air bonded by passing it around a honeycomb drier, which is a rotating drum adapted to suck hot air through the web. The temperature of the heated air was 340°F (171°C) at the heater (a burner), and the recycled air had cooled to 200°F (93°C) as it entered the inlet side of the heater. Exposure time to the heated air was about 9 seconds. Certain physical properties of the fabric are displayed in Table I:

TABLE I

| | |
|---|---|
| Weight, oz/yd$^2$ (g/m$^2$) | 2.2 (74.6) |
| Density, g/cm$^3$ | 0.059 |
| Tensile, MD, lb/in (kg/cm) | 6.0 (1.07) |
| Tensile, CD lb/in (kg/cm) | 0.9 (0.16) |
| Absorbent capacity,[1] GAT, % | 1840 |

[1] Absorbent capacity measured on entire fabric, including veneer. Absorbent capacity, as used herein, is the weight of deionized or distilled water absorbed, divided by the dry weight of the sample, multiplied by 100, as measured by a gravimetric absorbency tester ("GAT"), using a point source with the sample held on a horizontal plate. The sample was unloaded, i.e., no compression load was held on the sample. The GAT is described in detail in US—A—4 357 827. Briefly, the GAT is an apparatus for determining the weight of liquid flowing to or from a test site. The apparatus comprises, in combination:

A vessel for containing liquid, said vessel being supported solely by weighing means;

Indicating means for indicating the weight sensed by said weighing means;

A test surface to receive a specimen to be tested, said test surface including said test site;

Conduit means operatively connecting said vessel to said test site for directing a flow of liquid between said vessel and said test site; and

Means for vertically positioning said test site.

The fabric of Example 1 was incorporated into a catamenial device and specifically the panty shield 42 illustrated in Fig. 2. The fabric comprised a layer 44 of absorbent material (i.e. the fabric of Example 1) which is to be placed against the body for absorbing body fluid. It will be understood that while a single ply of fabric is illustrated, the absorbent layer of fabric may be multiplied by using several sheets of fabric or by folding a sheet upon itself. A barrier film, layer 46, which may consist of a fluid impermeable film, such as polyethylene is laminated to the absorbent material. Pressure-sensitive adhesive element 48 is placed on the face of the barrier film for adhering the panty shield 42 to the crotch of an undergarment. To protect the pressure-sensitive element 48 prior to use, a removable protective strip 50 is applied. The panty shield incorporating the fabric of this invention gives excellent performance.

Example 2

Using an apparatus similar to that shown in Fig. 1, two veneered fabrics were made from wood pulp and Fiber A. The veneers were all Fiber A. Both fabrics were bonded by placing them in a 300°F (149°C) oven, supported on a screen, for 30 seconds. Table II describes the two fabrics, and Table III displays certain properties of the fabrics:

**0 070 164**

TABLE II

| | Run 1 | Run 2 |
|---|---|---|
| Core composition: | | |
| % Fiber A | 30 | 26 |
| % Wood pulp | 70 | 74 |
| Core weight, oz/yd² (g/m²) | 1.5 (50.9) | 1.5 (50.9) |
| Top veneer wt., oz/yd² (g/m²) | 0.35 (11.9) | 0.17 (5.8) |
| Bottom veneer, wt., oz/yd² (g/m²) | 0.16 (5.4) | 0.17 (5.8) |

TABLE III

| | Run 1 | Run 2 |
|---|---|---|
| Weight, oz/yd² (g/m²) | 2.1 (71.2) | 2 (67.8) |
| Thickness, inch (cm) | 0.094 (0.24) | 0.091 (0.23) |
| Density, gm/cc | 0.030 | 0.029 |
| Tensile, lb/in (kg/cm) | | |
| MD | 3.0 (0.54) | 2.2 (0.39) |
| CD | 0.65 (0.12) | 0.45 (0.08) |
| Absorbency capacity, GAT, % | 2466 | 2299 |

Example 3

Using an arrangement of apparatus similar to that shown in Fig. 1 (with slight differences, as discussed below), a fabric designed for use as a plateroom wipe (a plateroom wipe is used in the offset printing industry to clean the printing plates and to apply developing emulsion) was produced as follows:

The fabric comprised a core with two veneers. Each veneer weighed 0.3 ounce per square yard (10.2 g/m²) and the core weighed 1.86 ounces per square yard (63.1 g/m²). The compositions of each layer were as follows:

TABLE IV

| Top veneer | | |
|---|---|---|
| 0.24 oz/yd² (8.1 g/m²) | Fiber A |
| 0.06 oz/yd² (2.0 g/m²) | Rayon A[2] |
| | |
| Core | | |
| 0.43 oz/yd² (14.6 g/m²) | Polyester A[3] |
| 0.43 oz/yd² (14.6 g/m²) | Fiber A |
| 1.00 oz/yd² (33.9 g/m²) | Pulp |
| | |
| Bottom veneer | | |
| 0.24 oz/yd² (8.1 g/m²) | Fiber A |
| 0.06 oz/yd² (2.0 g/m²) | Rayon A[2] |

[2] Avtex 1913 rayon staple fiber having a staple length of 1-1/8 inches (2.86 cm) and a denier of 1.5 (0.17 Tex).

[3] "Fortrel" 317 polyester staple fiber having a staple length of 1.5 inches (3.81 cm) and a denier of 1.5 (0.17 Tex).

5

The bottom veneer was laid down as shown in Fig. 1. The core was laid down by a dual rotor, with the two staple fibers being fed to the dual rotor by two cards, and the pulp being fed in the form of pulp board. The top veneer was then laid down, as shown in Fig. 1. The web was through-air bonded by the honeycomb drier described in Example 1. The temperature of the heated air was 160°—190°C. Exposure time to the heated air was about 2±1/2 seconds. After through-air bonding, the fabric was hot embossed by passing the fabric through the nip of a pair of counterrotating rolls, one roll being smooth and the other having a raised diamond pattern composed of two sets of raised parallel lines that intersected each other. Each set of lines was angled 30° from the machine direction to form a pattern of diamond-shaped parallelograms, each having two 60° angles and two 120° angles. Each line was 0.8 millimeter wide, 0.035 millimeter high, and the space between the lines (edge-to-edge) was 5 millimeters. The temperature of the rolls was 260°F (127°C), and the rolls were pressed together by a pressure cylinder operating at a pressure of 35 to 60 psig (2.4 to 4.1 bar). The cylinder had a diameter of 2 centimeters, and the width of the web was about 17 inches (43.2 cm).

A plateroom wipe 52 made from the fabric thus produced is shown in Figs. 3 and 4. The figures show the embossed lines 54 and the essentially three-layer construction comprising two veneers 56, 58 and a core 60.

The fabric described in this Example makes an excellent plateroom wipe for the following reasons:

a) It is soft and conformable;

b) It is absorbent;

c) It exhibits low linting;

d) It has high loft and bulk and retains these properties when wet;

e) While high strength is not required, the fabric retains adequate integrity and abrasion resistance during use;

f) The surface is not abrasive;

g) The fabric resists attack by the solvents, cleaning emulsions, and other liquids encountered in use;

h) The fabric is dimensionally stable; and

i) The embossed areas provide a place for dirt to collect. (The embossing also adds strength and integrity).

Some representative properties of the fabric described in Example 3 are shown below in Table V. The three samples were taken from different places during an extended run of the fabric.

# 0 070 164

TABLE V

| Sample No. | 1 | 2 | 3 |
|---|---|---|---|
| Weight—oz/yd² (g/m²) | 2.25 (76.3) | 2.26 (76.6) | 2.26 (76.6) |
| Bulk—inch (cm) | 0.046 (0.12) | 0.053 (0.13) | 0.057 (0.14) |
| Density—gm/cc | 0.065 | 0.056 | 0.053 |
| Dry tensile—lbs/in (kg/cm) | | | |
| MD | 3.54 (0.63) | 3.46 (0.62) | 4.58 (0.82) |
| CD | 0.59 (0.11) | 0.53 (0.09) | 1.59 (0.28) |
| Dry elongation—% | | | |
| MD | 40 | 42 | 37 |
| CD | 38 | 48 | 49 |
| Wet tensile—lbs/in (kg/cm) | | | |
| MD | 3.58 (0.64) | 3.48 (0.62) | 4.17 (0.75) |
| CD | 0.73 (0.15) | 0.70 (0.13) | 1.51 (0.27) |
| Wet elongation—% | | | |
| MD | 37 | 43 | 38 |
| CD | 62 | 51 | 53 |
| Absorbent capacity,[4][6] GAT—% | 829 | 912 | — |
| Handle-O-meter[4][5] | — | 157 | — |
| Bulk—inch (cm) | | | |
| Dry[4] | 0.043 (0.11) | — | — |
| Wet[4] | 0.039 (0.10) | — | — |

[4] Tested on different samples from the same run.
[5] Handle-O-Meter softness (in grams), tested on a 7-inch (17.8 cm) square sample using a 3/8-inch (0.95 cm) slot. Machine directions is perpendicular to slot.
[6] The GAT used a porous plate to hold the sample instead of a point source, and the test liquid was 1% saline solution.

Example 4

A fabric designed for use in wound dressings or eye pads was produced by an arrangement of apparatus as shown in Fig. 1. The fabric had a core layer of pulp plus staple fiber and one veneer layer. The composition of the fabric was as follows:

TABLE VI

| | |
|---|---|
| Core | |
| Fiber A | 0.5 oz/yd² (16.9 g/m²) |
| Rayon A | 1.0 oz/yd² (33.9 g/m²) |
| Pulp | 1.0 oz/yd² (33.9 g/m²) |
| Veneer | |
| Fiber A | 0.5 oz/yd² (16.9 g/m²) |
| Total weight | 3.0 oz/yd² (101.6 g/m²) |

7

The fabric was made by a procedure analogous to that described in Example 3, except that no embossing was carried out. The temperature and dwell time in the honeycomb drier were about 160°—200°C and about 3 seconds.

The fabric of this Example 4 was fabricated into a wound dressing 62, as shown in Figs. 5, 6, and 7. The dressing was a three-layer structure made by superimposing three layers of fabric, and then sonically bonding the layers around the edges. The two outer layers 64, 66 were the fabric described above in this Example 4 (veneer sides 68,70 out, as is shown in Fig. 7), with an inner layer 72 composed of a 1.5 oz/yd$^2$ (50.981 gm$^2$) through-air bonded web of 33% Fiber A and 67% Avtex 1913 rayon staple fibers.

The wound dressings made from the fabrics of this Example 4 exhibit the following desirable properties:

a) Good absorbent capacity; and

b) Ability to utilize a high proportion of the theoretical absorbent capacity of the dressing because of good horizontal wicking properties and the ability of the exterior face of the dressing to delay strike-through of aqueous liquids.

Table VII, below, displays some representative properties of the wound dressing described above:

TABLE VII

| | |
|---|---|
| Weight, oz/yd (g/m$^2$) | 7.8 (264) |
| Bulk, inch (cm) | 0.206 (0.52) |
| Absorbent capacity, GAT.%[7] | 1300 |
| Absorbency rate, GAT, g/min[8] | 21.4 |
| Strike-through time, seconds[9] | 14 |

[7] Test fluid was 1% saline with a red dye.
[8] The rate of liquid uptake in the GAT.
[9] The time for the test fluid to penetrate the exterior or top surface in the GAT test.

**Claims**

1. An absorbent, thermal bonded nonwoven fabric having a bulk density below about 0.15 gram per cubic centimeter, said fabric comprising pulp length absorbent fibers and staple length conjugate fibers, said conjugate fibers being composed of polyethylene and polyester, wherein a substantial proportion of the surfaces of said conjugate fibers comprises said polyethylene.

2. The nonwoven fabric of claim 1 wherein the bulk density of said fabric is within the range of from about 0.02 to about 0.09 gram per cubic centimeter.

3. The nonwoven fabric of claim 1 or 2 wherein said fabric has an absorbent capacity of at least about 1400 per cent.

4. The nonwoven fabric of any one of claims 1 to 3 wherein the conjugate fibers are sheath/core bicomponent fibers.

5. The nonwoven fabric of any one of claims 1 to 4 wherein at least one surface of said fabric has a veneer of thermal bonded heat-fusible fibers.

6. The nonwoven fabric of claim 5 wherein said heat-fusible fibers are conjugate fibers.

7. The nonwoven fabric of any one of claims 1 to 6 wherein said absorbent fibers are wood pulp fibers.

8. The nonwoven fabric of any one of claims 1 to 6 wherein the absorbent fibers are rayon staple fibers.

9. The nonwoven fabric of any one of claims 1 to 8 wherein said fabric further comprises staple length polyester fibers.

10. The nonwoven fabric of any one of claims 1 to 9 wherein said fabric is hot embossed.

11. Process which comprises:

(a) producing a web comprising pulp length absorbent fibers and staple length conjugate fibers, said conjugate fibers being composed of polyethylene and polyester, wherein a substantial proportion of the surface of said conjugate fibers comprises said polyethylene;

(b) subjecting said web to temperature sufficient to fuse said polyethylene without fusing said polyester, while maintaining said web under minimal pressure; and

(c) cooling said web to re-solidify said polyethylene, to thereby produce a low density, highly absorbent, thermal bonded nonwoven fabric.

12. Process of claim 11 wherein said web includes at least one veneer layer comprising heat-fusible fibers.

13. Process of claim 11 or 12 wherein the nonwoven fabric product of step (c) is subjected to the additional step of hot embossing.

14. A catamenial device comprising the nonwoven fabric of any one of claims 1 to 10.

8

15. The catamenial device of claim 14 comprising the nonwoven fabric of any one of claims 1 to 10 having laminated thereto an impermeable layer, said impermeable layer having applied thereon a pressure-sensitive adhesive element for adhering to an undergarment, said pressure-sensitive element having a protective layer to protect the same prior to use.

**Patentansprüche**

1. Ein absorbierender, durch Wärmeeinwirkung verbundenen Faservliesstoff mit einer Raumdichte von weniger als etwa 0,15 g/cm³, wobei der genannte Stoff absorbierende Fasern von Zellstoffaserlänge und konjugierte Fasern von Stapelfaserlänge umfaßt, wobei die genannten konjugierten Fasern aus Polyethylen und Polyester zusammengesetzt sind und wobei ein wesentlicher Anteil der Oberflächen der genannten konjugierten Fasern Polyethylen enthält.

2. Der Faservliesstoff nach Anspruch 1, worin die Raumdichte des genannten Stoffes im Bereich von etwa 0,02 bis etwa 0,09 g/cm³ liegt.

3. Der Faservliesstoff nach Anspruch 1 oder 2, worin der genannte Stoff ein Absorptionsvermögen von wenigstens etwa 1400% aufweist.

4. Der Faservliesstoff nach einem der Ansprüche 1 bis 3, worin die konjugierten Fasern von Stapelfaserlänge Kern/Mantel-Zweikomponentenfasern sind.

5. Der Faservliesstoff nach einem der Ansprüche 1 bis 4, worin wenigstens eine Oberfläche des genannten Stoffes eine Deckschicht aus durch Wärmeeinwirkung verbundenen, unter Wärmeeinwirkung schmelzbaren Fasern aufweist.

6. Der Faservliesstoff nach Anspruch 5, worin die genannten unter Wärmeeinwirkung schmelzbaren Fasern konjugierte Fasern sind.

7. Der Faservliesstoff nach einem der Ansprüch3 1 bis 6, worin die genannten absorbierenden Fasern Holzzellstoffasern sind.

8. Der Faservliesstoff nach einem der Ansprüche 1 bis 6, worin die absorbierenden Fasern Reyon-Stapelfasern sind.

9. Der Faservliesstoff nach einem der Ansprüche 1 bis 8, worin der genannte Stoff zusätzlich Polyesterfasern von Stapelfaserlänge umfaßt.

10. Der Faservliesstoff nach einem der Ansprüche 1 bis 9, worin der genannte Stoff unter Wärmeeinwirkung geprägt worden ist.

11. Ein Verfahren, welches die folgenden Stufen umfaßt;

(a) die Erzeugung einer absorbierende Fasern von Zellstofffaserlänge und konjugierte Fasern von Stapelfaserlänge umfassenden Bahn, wobei die genannten konjugierten Fasern aus Polyethylen und Polyester zusammengesetzt sind und wobei ein wesentlicher Anteil der Oberflächen der genannten konjugierten Fasern das genannte Polyethylen enthält;

(b) das Einwirkenlassen einer Temperatur, welche zum Schmelzen des genannten Polyethylens ausreicht, ohne den genannten Polyester zu schmelzen, auf die Bahn, während dieselbe unter einem möglichst niedrigen Druck gehalten wird;

(c) das Abkühlen der gennanten Bahn zum Wiederverfestigen des genannten Polyethylens, um dadurch einen hochgradig absorbierenden, durch Wärmeeinwirkung verbundenen Faservliessoff von niedriger Dichte zu erzeugen.

12. Verfahren nach Anspruch 11, worin die genannte Bahn wenigstens eine Deckschicht, welche unter Wärmeeinwirkung schmelzbare Fasern umfaßt, enthält.

13. Verfahren nach Anspruch 11 oder 12, worin das Faservliesstoffprodukt aus Stufe (c) der zusätzlichen Stufe eines Prägens unter Wärmeeinwirkung unterworfen wird.

14. Ein Monatshygieneartikel, umfassend den Faservliesstoff nach einem der Ansprüche 1 bis 10.

15. Der Monatshygieneartikel nach Anspruch 14, umfassend den Faservliesstoff nach einem der Ansprüche 1 bis 10, auf welchen Faservliesstoff ein undurchlässige Schicht auflaminiert ist, wobei auf die genannte undurchlässige Schicht ein druckempfindliches Klebeelement zum Ankleben des Artikels an einem Unterwäschestück aufgebracht ist, wobei das genannte druckempfindliche Element mit einer Schutzchicht bedeckt ist, welche das Element vor dem Gebrauch schützt.

**Revendications**

1. Etoffe absorbante non tissée, thermiquement liée, ayant une masse volumique apparente inférieure à environ 0,15 g/cm³, ladite étoffe comprenant des fibres absorbantes de longueur de pâte et des fibres coupées conjuguées, lesdites fibres conjuguées étant composées de polyéthylène et de polyester, une proportion notable des surfaces desdites fibres conjuguées comprenant ledit polyéthylène.

2. Etoffe non tissée selon la revendication 1, dans laquelle la masse volumique apparente de ladite étoffe est comprise entre environ 0,02 et 0,09 g/cm³.

3. Etoffe non tissée selon la revendication 1 ou 2, dans laquelle ladite étoffe possède une capacité absorbante d'au moins 1400% environ.

4. Etoffe non tissée selon l'une quelconque des revendications 1 à 3, dans laquelle les fibres conjuguées sont des fibres à deux composants gaine/noyau.

5. Etoffe non tissée selon l'une des revendications 1 à 4, dans laquelle au moins une surface de ladite étoffe comporte un revêtement en fibres fusibles par la chaleur thermiquement liées.

6. Etoffe non tissée selon la revendication 5, dans laquelle lesdites fibres fusibles par la chaleur sont des fibres conjuguées.

7. Etoffe non tissée selon l'une quelconque des revendications 1 à 6, dans laquelle lesdites fibres absorbantes sont des fibres de pâte de bois.

8. Etoffe non tissée selon l'une quelconque des revendications 1 à 6, dans laquelle les fibres absorbantes sont des fibres coupées de rayonne.

9. Etoffe non tissée selon l'une quelconque des revendications 1 à 8, dans laquelle ladite étoffe comprend en outre des fibres coupées en polyester.

10. Etoffe non tissée selon l'une quelconque des revendications 1 à 9, dans laquelle ladite étoffe est gaufrée à chaud.

11. Procédé qui comprend les étapes consistant:

(a) à produire un tissu comprenant des fibres absorbantes de longueur de pâte et des fibres conjuguées coupées, lesdites fibres conjuguées étant composées de polyéthylène et de polyester, une proportion notable de la surface desdites fibres conjuguées comprenant ledit polyéthylène;

(b) à soumettre ledit tissu à une température suffisante pour fondre ledit polyéthylène sans fondre ledit polyester pendant qu'on maintient ledit tissu sous une pression minimale; et

(c) à refroidir ledit tissu pour resolidifier ledit polyéthylène pour obtenir ainsi une étoffe non tissée, thermiquement liée, hautement absorbante et de faible densité.

12. Procédé selon la revendication 11, dans lequel ledit tissu comprend au moins une couche de revêtement comprenant des fibres fusibles par la chaleur.

13. Procédé selon la revendication 11 ou 12, dans lequel on soumet l'étoffe non tissée du stade (c) au stade supplémentaire de gaufrage à chaud.

14. Dispositif cataménial comprenant l'étoffe non tissée de l'une quelconque des revendications 1 à 10.

15. Dispositif cataménial selon la revendication 14, comprenant l'étoffe non tissée de l'une quelconque des revendications 1 à 10 sur laquelle est stratifiée une couche imperméable, ladite couche imperméable portant un élément adhésif sensible à la pression pour adhérer à un sous-vêtement, ledit élément sensible à la pression ayant une couche protectrice pour le protéger avant utilisation.

Fig.1.

Fig.2.

0 070 164

Fig.3.

52

54

Fig.4.

52

56

60

54

58

Fig.5.

7

62

7

Fig.6.

62

64

72

66

Fig.7.

64

68

72

66

70